# EUROPEAN PATENT APPLICATION

(11) **EP 3 476 335 A1**
(43) Date of publication of application: **01.05.2019**
(21) Application number: 17819047.6
(22) Date of filing: 06.06.2017
(51) Int. Cl.: A61B 17/32

(54) **TOOL BIT FOR PIEZOSURGERY**

(30) Priority: 27.06.2016 CN 201620643862 U
(71) Applicant: Jiangsu SMTP Technology Co., Ltd., Zhangjiagang, Jiangsu 215634 (CN)
(72) Inventor: SUN, Chuiguo, Zhangjiagang Jiangsu 215634 (CN); CHEN, Zhongqiang, Zhangjiagang Jiangsu 215634 (CN); LI, Li, Zhangjiagang Jiangsu 215634 (CN); ZHAN, Songtao, Zhangjiagang Jiangsu 215634 (CN); CAO, Qun, Zhangjiagang Jiangsu 215634 (CN)
(74) Representative: Balder IP Law, S.L.
(86) International application number: PCT/CN2017/087243
(87) International publication number: WO 2018/001050

(57) **Abstract**

Disclosed is a tool bit for an ultrasonic osteotome comprising a cutting portion (1) at a front end of the tool bit for an ultrasonic osteotome, a bit body (3) connected to a transducer, and an arbor (2) connecting the cutting portion (1) and the bit body (3), wherein the cutting portion (1) is of a flat sheet structure, two narrow faces of which form an upper cutter ridge (4) and a lower cutter ridge (5) of the cutting portion (1), a length of the upper cutter ridge (1) being greater than that of the lower cutter ridge(1).The upper cutter ridge (4) and the lower cutter ridge (5) are connected by an arc portion at a head end of the cutting portion (1), and the arc portion (1) is formed with cutter teeth (6). The cutting portion (1) is bent in a direction towards the upper cutter ridge (4). The upper cutter ridge (4) and the lower cutter ridge (5) are parallel flat surfaces, and a bent portion is formed at a position on the arbor adjacent to the cutting portion (1). The use of the tool bit for an ultrasonic osteotome of the present invention in a posterior spinal surgery for vertebral body resection can help a doctor bypass the spinal cord for longitudinal cutting and osteotomy in a small space, safely complete the operation without injuring the spinal cord.

## Description

### TECHNICAL FIELD

The present disclosure relates to the field of medical devices, in particular to a scalpel, and more particularly to a tool bit for an ultrasonic osteotome.

### BACKGROUND OF THE INVENTION

In modern society, with the development of medical technology, orthopedic surgery shows a trend of diversity. Accordingly, when performing a surgery, it is necessary to use different tool bits for a scalpel to perform cutting, grinding, scraping, clamping and other operations on an affected part of a patient accordi ng to different orthopedic conditions of disease.

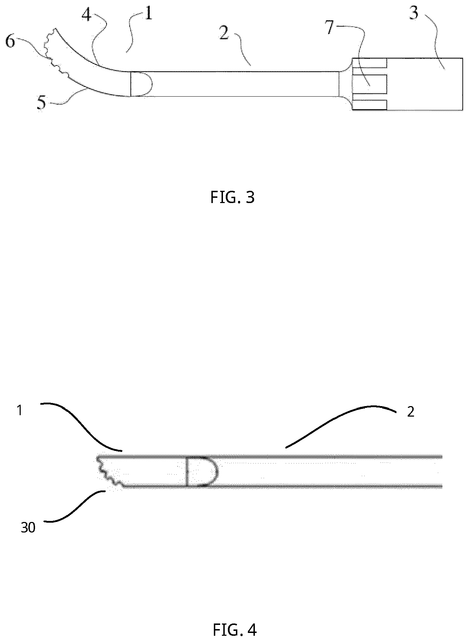

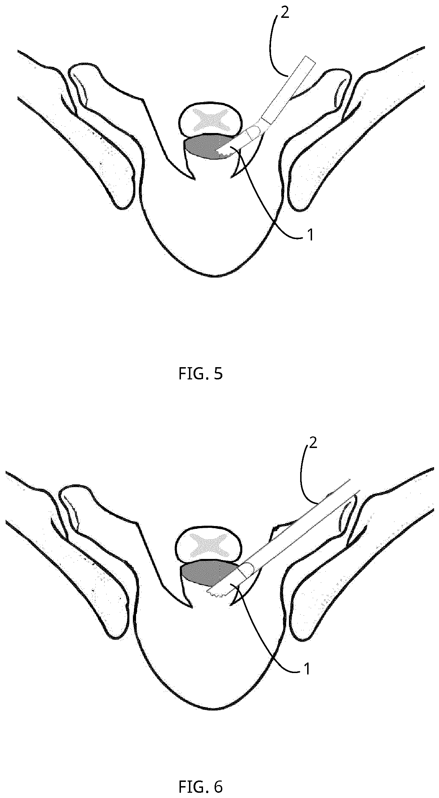

In view of the special construction of bone structure, along with the continuously development of ultrasound technology in recent years, an ultrasonic osteotome has gradually become a main tool for modern orthopedic surgery. In orthopedic surgery, a commonly used blade-shaped tool bit for an ultrasonic osteotome is a tool bit mainly used for cutting, but at present, such tool bits are mostly straight-shaped, as shown in Fig.4. When it is necessary to bypass a spinal cord to cut a posterior portion of a vertebral body during an osteotomy operation, a large operating space is required to allow the tool bit to enter the front of the spinal cord, as shown in Fig. 6. In this way, it is extremely inconvenient for operation, and it is prone to cause medical accidents due to operational errors.

### SUMMARY OF THE INVENTION

In view of the problems existing in the prior art, the present disclosure provides a tool bit for an ultrasonic osteotome comprising a cutting portion at a front end of the tool bit for an ultrasonic osteotome, a bit body connected to a transducer, and an arbor connecting the cutting portion and the bit body, wherein the cutting portion is of a flat sheet structure, two narrow faces of which form an upper cutter ridge and a lower cutter ridge of the cutting portion, a length of the upper cutter ridge being greater than that of the lower cutter ridge. The upper cutter ridge and the lower cutter ridge are connected by an arc portion at a head end of the cutting portion, and the arc portion is formed with cutter teeth. The cutting portion is bent in a direction towards the upper cutter ridge, the upper cutter ridge and the lower cutter ridge are parallel flat surfaces, and a bent portion is formed at a position on the arbor adjacent to the cutting portion.

According to an embodiment of the tool bit for an ultrasonic osteotome of the present invention, it is preferable that the bent portion adopts an arc transition.

According to an embodiment of the tool bit for an ultrasonic osteotome of the present invention, it is preferable that the upper cutter ridge and the lower cutter ridge are curved in the direction of the upper cutter ridge with a same curvature to form an arc-shaped upper cutter ridge and lower cutter ridge.

According to an embodiment of the tool bit for an ultrasonic osteotome of the present invention, preferably, an outer diameter of the arbor is smaller than an outer diameter of the bit body, and the arbor and the bit body adopt a bevel transition.

According to an embodiment of the tool bit for an ultrasonic osteotome of the present invention, preferably, an outer diameter of the arbor is smaller than an outer diameter of the bit body, and the arbor and the bit body adopt an arc transition.

According to an embodiment of the tool bit for an ultrasonic osteotome of the present invention, preferably, a threaded structure is provided at a tail end of the bit body.

According to an embodiment of the tool bit for an ultrasonic osteotome of the present invention, preferably, a thread of the bit body is an internal thread.

According to an embodiment of the tool bit for an ultrasonic osteotome of the present invention, it is preferable that the bit body is provided with clamping faces for clamping.

According to an embodiment of the tool bit for an ultrasonic osteotome of the present invention, it is preferable that the number of the clamping faces is an even number and the clamping faces are symmetrically arranged in pairs.

A nother aspect of the present invention provides a tool bit for an ultrasonic osteotome comprising a cutting portion at a front end of the tool bit for an ultrasonic osteotome, a bit body connected to a transducer, and an arbor connecti ng the cutting portion and the bit body, wherein the cutti ng portion is of a flat sheet structure, two narrow faces of which form an upper cutter ridge and a lower cutter ridge of the cutting portion, a length of the upper cutter ridge being greater than a length of the lower cutter ridge. The upper cutter ridge and the lower cutter ridge are connected by an arc portion at a head end of the cutting portion, and the arc portion is formed with cutter teeth, the cutting portion is bent in a direction of the upper cutter ridge, and the upper cutter ridge and the lower cutter ridge are curved in the direction towards the upper cutter ridge with a same curvature to form an arc-shaped upper cutter ridge and lower cutter ridge.

The tool bit for an ultrasonic osteotome of the invention is simple in structure, convenient for machining, flexible in operation and of wide range of use. The use of the tool bit for an ultrasonic osteotome of the present invention in the posterior spinal surgery for vertebral body resection can help a doctor bypass the spinal cord for longitudinal cutting and osteotomy in a small space, safely complete the operation without injuring the spinal cord. The tool bit can also perform osteotomy operati on instead of a straight-shaped tool bit, but the space required is smaller, which reduces a length of a surgical incision, reduces the risk of surgery, and shortens the operati on time.

### BRIEF DESCRIPTION OF THE DRAWINGS

In order to more clearly illustrate the technical sol utions in specific embodiments of the present invention or in the prior art, the drawings used in the description of the specific embodiments or the prior art will be briefly described below. Apparently, the drawings described in the following description are only some embodiments of the present invention, and those skilled in the art can obtain other drawings based on these drawings without creative work.
Fig. 1 is a perspective view of a tool bit for an ultrasonic osteotome according to a first embodiment of the present invention,
Fig. 2 is a front elevational view showing the tool bit for an ultrasonic osteotome according to the first embodiment of the present invention,
Fig. 3 is a front elevational view showing a tool bit for an ultrasonic osteotome according to a second embodiment of the present invention,
Fig. 4 is a front elevational view showing a straight-shaped tool bit for an ultrasonic osteotome in the prior art,
Fig. 5 is a schematic view showing a state of a bone tissue when a tool bit for an ultrasonic osteotome of the present invention is used to perform an operation,
Fig. 6 is a schematic view showing a state of a bone tissue when a tool bit for an ultrasonic osteotome in the prior art is used to perform an operation.

### Reference Numerals:

1- cutting portion, 2-arbor, 3-bit body, 4-upper cutter ridge, 5-lower cutter ridge, 6-cutter teeth, 7-clamping face.

### DETAILED DESCRIPTION OF THE INVENTION

Embodiments of the present disclosure will be described herein after clearly and completely with reference to accompanying drawings. Apparently, the embodiments described herein are only portions of embodiments of the invention, rather than all embodiments of the invention. It is intended that all other embodiments obtained by those skilled in the art based on the disclosed embodiments without inventive labor are within the scope of the present invention.

In the description of the present disclosure, it is to be noted that the terms of 'center_, "upper_, lower_, 'left_, 'right_, 'vertical_, 'horizontal_, 'internal_, 'external_ and the like simply indicate orientational or positional relationship based on the accompanying drawings and are used only for the purpose of facilitating and simplifying the description of the invention, rather than specifying or implying that any device or elements indicated must have a certain orientation, consti tute with a certain orientation, or operate in a certain orientation. Therefore, these terms will not be interpreted as limiting the present invention. Further, the terms of 'first_, "second_ and 'third_ are only used for description purpose, rather than being interpreted as specifying or implying relative importance.

In the description of the present disclosure, it is to be noted that, unless otherwise specified or defined clearly, the terms of 'attach_, 'connectto_, 'connect with_, 'couple_ and the like should be interpreted broadly. For example, they may refer to fixed connection, or detachable connection, or integral connection; they may refer to mechanical connection, or electrical connection, they may refer to direct connection, or indirect connection through an intermediate media, or internal communication between two components. For those skilled in the art, the specific meaning of these terms in the present disclosure may be understood in combination with specific situations.

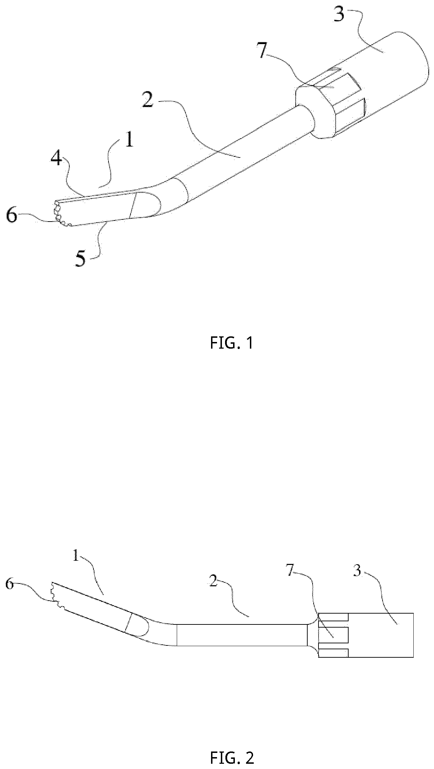

Fig. 1 is a perspective view of a tool bit for an ultrasonic osteotome according to a first embodiment of the present invention. As shown in Fig. 1, the tool bit for an ultrasonic osteotome according to the first embodiment of the present invention comprises a cutting portion 1 at a front end of the tool bit for an ultrasonic osteotome, a bit body 3 connected to a transducer, and an arbor 2 with one end connected to the cutting portion 1 and the other end connected to the bit body 3. The cutting portion 1 is of a flat plate structure, and two narrow faces of the flat plate structure form an upper cutter ridge 4 and a lower cutter ridge 5 of the cutting portion 1. A length of the upper cutter ridge 4 is larger than that of the lower cutter ridge 5. The upper cutter ridge 4 and the lower cutter ridge 5 are connected by an arc portion at a head end of the cutting portion 1. The arc portion is formed with cutter teeth 6 thereon. The cutting portion 1 is bent in a direction of the upper ridge 4.

When a vertebral body resection is performed in a posterior spinal surgery, the tool bit for an ultrasonic osteotome of the present invention can help a doctor bypass a spinal cord for longitudinal cutting and osteotomy in a small space, and safely complete the operation without injuring the spinal cord.

Fig. 2 is a front elevational view showing the tool bit for an ultrasonic osteotome according to the first embodiment of the present invention. As shown in Fig. 1 and Fig. 2, in the tool bit for an ultrasonic osteotome according to the first embodiment of the present invention, the upper cutter ridge 4 and the lower cutter ridge 5 are parallel flat surfaces, and a bent portion is located on a position on the arbor 2 adjacent to the cutting portion 1. The doctor can choose tool bits for an ultrasonic osteotome of different configurations according to the necessity of operation.

In the tool bit for an ultrasonic osteotome according to the first embodiment of the present invention, the bent portion may adopt an arc transition. An arc transition is easy for machining and shaping, and a transition portion can be smoothed at a low cost, and an operator and a surgical patient can be well protected.

Fig. 3 is a front elevational view of a tool bit for an ultrasonic osteotome according to a second embodiment of the present invention. A s shown in Fig. 3, an upper cutter ridge 4 and an lower cutter ridge 5 of the tool bit for an ultrasonic osteotome accordi ng to the second embodiment of the present invention are curved in a di rection towards the upper cutter ridge 4 with a same curvature, so as to form an arc-shaped upper cutter ridge 4 and lower cutter ridge 5. This meniscus shaped tool bit for an ultrasonic osteotome has a more suitable shape for bypassing a cylindrical tissue and operating behind it.

In the tool bit for an ultrasonic osteotome according to the second embodiment of the present invention, an outer diameter of an arbor 2 may be smaller than an outer di ameter of a bit body 3, and the arbor 2 and the bit body 3 may adopt a bevel transition. The tool bit for an ultrasonic osteotome of this shape is more compact in structure and more convenient to use.

Moreover, in the tool bit for an ultrasonic osteotome according to the second embodiment of the present invention, an outer diameter of the arbor 2 may be smaller than an outer diameter of the bit body 3, and the arbor 2 and the bit body 3 may adopt an arc transition. The arc transition has a smooth structure, avoiding stress concentration on the one hand, and having a good protection effect for operators and surgical patients on the other hand.

In the tool bit for an ultrasonic osteotome according to the first and second embodiments of the present invention, the bit body 3 may be provided with a threaded structure at a tail end thereof, and preferably, a thread of the bit body 3 is an internal thread. The threaded structure is a common way of detachable connection, but the tool bit for an ultrasonic osteotome of the present invention is not limited to the manner of threaded connecti on. Instead, any structure can be used as a connection structure of the tool bit for an ultrasonic osteotome of the present invention, as long as it can connect the tool bit for an ultrasonic osteotome of the present invention to a transducer detachably, such as a snap-fit structure, or the like.

In the tool bit for an ultrasonic osteotome according to the first and second embodiments of the present invention, the bit body 3 may be provide with clamping faces 7 for clamping, which can facilitate an operator to screw the tool bit for an ultrasonic osteotome of the present invention onto a transducer with a tool, thereby improving its work reliability. Preferably, the number of clamping faces 7 is an even number and the clamping faces 7 are symmetrically arranged in pairs.

Compared with the prior art, the tool bit for an ultrasonic osteotome of the invention is simple in structure, convenient for machining, flexible for operation and of wide range of use. As shown in Fig. 5, when the tool bit for an ultrasonic osteotome of the present invention is used to perform a vertebral body resection in a posterior spinal surgery, it can help a doctor bypass a spinal cord for longitudinal osteotomy cutti ng operation in a small space, so that the operation can be safely completed without injuring the spinal cord. This beneficial technical effect is difficult to achieve in a spinal surgery using a straight-shaped tool bit in the prior art as shown in Fig. 6.

In addition, the tool bit for an ultrasonic osteotome of the present invention can also be used to replace a straight-shaped tool bit for performing an osteotomy operation, but the space required is smaller, which reduces a length of a surgical incision, thus reducing risk of surgery, and shortening the operation time.

Finally, it should be noted that the above embodiments are only used to describe the sol utions of the present invention, rather than limiting the present invention. Although detailed descriptions of the invention are made with reference to the above embodiments, it would be appreciated by those skilled in the art that various changes or modifications to the above embodiments may be made or equivalent substitutions to portion of or all features in those embodiments may be made. Such changes, modifications or substitutions will not make the spirit of the relevant solutions depart from the scope of the present invention.

## Claims

1. A tool bit for an ultrasonic osteotome, comprising a cutting portion at a front end of the tool bit for an ultrasonic osteotome, a bit body connected to a transducer, and an arbor connecting the cutting portion and the bit body, wherein
the cutting portion is of a flat sheet structure, two narrow faces of which form an upper cutter ridge and a lower cutter ridge of the cutting portion, and a length of the upper cutter ridge being greater than that of the lower cutter ridge,
the upper cutter ridge and the lower cutter ridge are connected by an arc portion at a head end of the cutti ng portion, and the arc portion is formed with cutter teeth,
the cutting portion is bent in a direction towards the upper cutter ridge, the upper cutter ridge and the lower cutter ridge are parallel flat surfaces, and a bent portion is formed at a position on the arbor adjacent to the cutting portion.

2. The tool bit for an ultrasonic osteotome according to claim 1, wherein
the bent portion adopts an arc transition.

3. The tool bit for an ultrasonic osteotome according to claim 1 or 2, wherein
an outer diameter of the arbor is smaller than an outer diameter of the bit body, and the arbor and the bit body adopt a bevel transition.

4. The tool bit for an ultrasonic osteotome according to claim 1 or 2, wherein
an outer diameter of the arbor is smaller than an outer diameter of the bit body, and the arbor and the bit body adopt an arc transition.

5. The tool bit for an ultrasonic osteotome according to claim 1 or 2, wherein
a threaded structure is provided at a tail end of the bit body.

6. The tool bit for an ultrasonic osteotome according to claim 5, wherein
a thread of the bit body is an internal thread.

7. The tool bit for an ultrasonic osteotome according to claim 1 or 2, wherein the bit body is provided with clamping faces for clamping.

8. The tool bit for an ultrasonic osteotome according to claim 7, wherein
the number of the clamping faces is an even number and the clamping faces are symmetrically arranged in pairs.

9. A tool bit for an ultrasonic osteotome comprisi ng a cutti ng portion at a front end of the tool bit for an ultrasonic osteotome, a bit body connected to a transducer, and an arbor connecting the cutting portion and the bit body, wherein
the cutting portion is of a flat sheet structure, two narrow faces of which form an upper cutter ridge and a lower cutter ridge of the cutting portion, and a length of the upper cutter ridge being greater than a length of the lower cutter ridge,
the upper cutter ridge and the lower cutter ridge are connected by an arc portion at a head end of the cutti ng porti on, and the arc portion is formed with cutter teeth,
the cutting portion is bent in a direction of the upper cutter ridge, and the upper cutter ridge and the lower cutter ridge are curved in the direction towards the upper cutter ridge with a same curvature to form an arc-shaped upper cutter ridge and lower cutter ridge.
